## Europäisches Patentamt
## European Patent Office
## Office européen des brevets

(19)

(11) Veröffentlichungsnummer: **0 120 112**
**B1**

# EUROPÄISCHE PATENTSCHRIFT

(12)

(45) Veröffentlichungstag der Patentschrift:
02.09.87

(21) Anmeldenummer: **83105060.4**

(22) Anmeldetag: **21.05.83**

(51) Int. Cl.⁴: **A 61 B 1/06**

(54) **Instrument zur Einführung von Narkosekathetern.**

(30) Priorität: **25.01.83 DE 8301892 U**

(43) Veröffentlichungstag der Anmeldung:
**03.10.84 Patentblatt 84/40**

(45) Bekanntmachung des Hinweises auf die Patenterteilung:
**02.09.87 Patentblatt 87/36**

(84) Benannte Vertragsstaaten:
**AT BE CH DE FR GB IT LI NL SE**

(56) Entgegenhaltungen:
DE-A-2 504 663
DE-A-2 926 919
GB-A-1 494 082
GB-A-2 092 892
US-A-2 649 087

(73) Patentinhaber: **Storz- Endoskop GmbH,
Kreuzgutweg 22, CH- 8207 Schaffhausen (CH)**

(72) Erfinder: **Storz, Karl, Auf dem Schildrain 39, D-7200
Tuttlingen (DE)**

(74) Vertreter: **Wenzel, Joachim, Dipl.- Ing.,
Hauptmannsreute 46, D-7000 Stuttgart 1 (DE)**

### Beschreibung

Die Neuerung betrifft ein Instrument zur Einführung von Narkosekathetern nach dem Oberbegriff des Anspruchs 1.

Es ist bereits ein flexibles Endoskop mit einem bildübertragenden flexiblen Element bekannt, das einen flexiblen Außentubus aufweist, wobei ein starres Kopfstück zwischen dem Außentubus und dem Okular vorgesehen ist. Hierbei ist unter anderem vorgesehen, daß das distale Ende mittels in dem Endoskopschaft angeordneten Zug - und/oder Federelementen in verschiedene Richtungen bewegbar ist. Solche Endoskope werden für Operationen in den höher gelegenen Darmwegen verwendet. Zur Betätigung der erwähnten Federelemente werden hierbei unter anderem Servo-Motoren im Kopfstück angeordnet, wodurch der Operateur wesentlich leichter manipulieren kann (DE-A-2 504 663).

Es ist weiter ein Instrument zur Einführung von Narkosekathetern in den Kehlkopf und die Luftröhre unter Sichtkontrolle mit einer langgestreckten gekrümmten Einführungssonde bekannt, die ebenfalls einen Glasfaser - Bildleiter enthält, wobei die Sonde von dem Narkosekatheter während der Einführung teilweise ummantelt wird. Um dieses Instrument wesentlich kürzer und somit handlicher, ganz unabhängig von der Größe des Patienten, zu gestalten, ist die starre Einführungssonde in diesem Falle mit mindestens einer mindestens teilweisen Schraubenwindung ausgebildet (DE-U-8 214 033.2). Hierbei ist das Okular an der Sonde starr befestigt, sodaß der Arzt große Mühe hat, der während des Einführens stark geänderten Einführungssrichtung der Sonde oder des Endoskopes zu folgen.

Der Erfindung liegt die Aufgabe zugrunde, diesen Nachteil zu beheben und die Möglichkeit zu schaffen, daß der Arzt der im Verlauf der Einführung sich ändernden Richtung des Laryngoskopes leicht zu folgen.

Zur Lösung dieser Aufgabe sind die kennzeichnenden Merkmale des Anspruchs 1 vorgesehen. Dadurch besteht die Möglichkeit, den Winkel zwischen dem Kopfstück und dem Okularstück auch während des Einführens des Laryngoskops ständig leicht zu ändern.

Gemäß einem weiteren Merkmale der Erfindung kann dieser Winkel sogar um mehr als 180° geändert werden. Da das bildübertragende flexible Element leicht biegbar und darüber hinaus mit entsprechender Überlänge ausgestattet ist, kann der Winkel sehr leicht von Hand geändert werden.

Weitere Vorteile und Einzelheiten der Neuerung ergeben sich aus der nun folgenden Beschreibung eines Ausführungsbeispiels unter Hinweis auf die Zeichnung. In dieser zeigen:

Fig. 1 eine Seitenansicht auf den Neuerungsgegenstand und

Fig. 2 eine Draufsicht auf einen Teil des Neuerungsgegenstandes nach Fig. 1

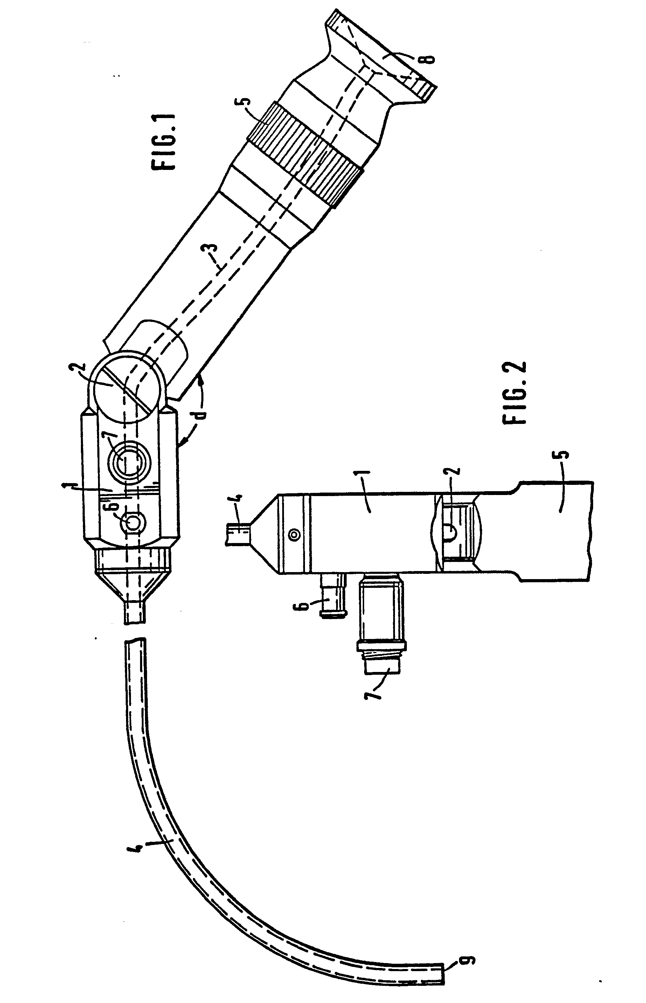

Fig. 1 zeigt ein Intubations-Laryngoskop mit einem Endoskopschaft 4, der weiter rechts an das Kopfstück 1 angeschlossen ist. Der Endoskopschaft 4 ist nur teilweise dargestellt. Er ist in Wahrheit sehr lang und zeigt über seine Länge eine erhebliche Biegung. Er besteht aus einem weich-biegsamen Material, das den Bedürfnissen anpassbar ist.

Das Kopfstück enthält einen Anschluß 6 für das Intubationsgas und einen weiteren Anschluß 7 weiter rechts für den beweglichen Lichtleiter.

Gemäß der Erfindung ist nun zwischen diesem Kopfstück 1 und dem Okular-Stück 5 ein Gelenk 2 angeordnet, um das das Okular-Stück 5 um einen Winkel $\alpha$ gegenüber dem Kopfstück 1 gemäß Fig. i geschwenkt ist. Am proximalen Ende des Okular-Stückes 5 befindet sich das Okular 8.

In dem Schaft 4 befindet sich in bekannter Weise ein Lichtleiter, der zu dem Anschluß 7 führt sowie der zu dem Anschluß 6 führende Gaskanal. Dies muß nicht im einzelnen dargestellt werden, weil es für sich bekannt ist. Ferner ist auch schon bekannt, daß der Endoskopschaft 4 darüber hinaus ein bildübertragendes, flexibles Element 3 enthält, welches das Bild zum Okular 8 überträgt. Dieses ist mit unterbrochenen Linien, weil hier nicht sichtbar, dargestellt. Gemäß der Neuerung ist vorgesehen, daß das bildübertragende flexible Element 3 zwischen dem Kopfstück 1 und dem Okular-Stück 5 eine gewisse Überlänge aufweist, damit das Element beim Schwenkeneum den Winkel $\alpha$ entsprechend nachgeben kann. Dabei kann das Scharnier 2 so ausgebildet sein, daß ein Schwenkwinkel $\alpha$ von mehr als 180° insgesamt möglich ist.

Fig. 2 zeigt die Draufsicht auf das Instrument nach Fig. 1. Man sieht hier vor allem, daß das Scharnier 2 in an sich bekannter Weise gabelförmig ausgebildet ist, was nicht in allen Einzelheiten dargestellt werden muß, weil Scharniere der verschiedensten Art dem Fachmann bestens bekannt sind. Die Bewegbarkeit des Scharnieres kann sehr leicht sein, da diese durch entsprechende Scharnier-Schrauben eingestellt werden kann. Das muß ebenfalls nicht in allen Einzelheiten angegeben werden, zumal hier auch bekannte Varianten bei Scharnieren möglich sind.

Zur Benutzung führt der Arzt zunächst das distale Ende 9 des Schaftes 4 in den Kehlkopf des Patienten unter Sichtbeobachtung durch das Okular 8. Beim weiteren Einschieben hat es der Arzt leicht, weil er den Winkel $\alpha$ beliebig von Hand ändern und somit der Biegung des starren Kopfstückes 1 leicht nachgeben kann. Dabei ändert sich das im Okular empfangene Bild 8 im wesentlichen nicht, auch wenn der Winkel $\alpha$ sehr stark geändert wird.

Durch die Erfindung wird somit die Manipulation des Arztes wesentlich erleichtert.

## Patentansprüche

1. Instrument zur Einführung von Narkosekathetern mit einem bildübertragenden flexiblen Element, einem flexiblen Lichtleiter und einem Kanal für das Intubationsgas, das ein Kopfstück (1) zur Aufnahme der Anschlüsse für das bildübartragende Element des flexiblen Lichtleiters (3) und das Gas enthält, wobei am patientenfernen Ende ein Okular (8) angeordnet ist, dadurch gekennzeichnet, daß zur Verbindung des Kopfstückes (1) mit einem Okular-Stück (5) am patientenfernen Ende ein gabelförmiges Scharnier (2) angeordnet ist und daß das bildübertragende flexible Element (3) zwischen dem Kopfstück und dem Okular (8) eine Überlänge aufweist.

## Claims

Instrument for inserting anesthesia catheters with an image-transmitting flexible element, a flexible light guide and a duct for the intubation gas, which contains a headpiece (1) for receiving connections for the image-transmitting element of the flexible light guide (3) and the gas, an eyepiece (8) being located at the patient-remote end, characterized in that a fork-shaped hinge (2) is provided for connecting the headpiece (1) to an eyepiece member (5) at the patient-remote end and that the image-transmitting flexible element (3) has an extra length between the headpiece and the eyepiece (8).

## Revendication

Instrument pour introduire des cathéters pour l'anesthésie, qui comporte un élément flexible transmettant l'image, un conducteur à fibre optique, et un canal pour le gaz d'intubation, et qui comprend également une pièce de tête (1) servant à recevoir les raccordements pour l'élément, transmettant l'image, du conducteur à fibre optique (3) flexible et pour le gaz, un oculaire (8) étant disposé à l'extrémité éloignée du patient, instrument caractérisé en ce que, pour relier la pièce de tête (1) à une pièce (5) associée à l'oculaire, une charnière (2) en forme de fourche est montée à l'extrémité éloignée du patient, et en ce que l'élément flexible (3) transmettant l'image comporte une surlongueur entre la pièce de tête et l'oculaire (8).

FIG.1

FIG.2

0120112